# EUROPEAN PATENT SPECIFICATION

(11) **EP 1 366 759 B1**
(45) Date of publication and mention of the grant of the patent: **03.06.2009**
(21) Application number: 02703917.1
(22) Date of filing: 05.03.2002
(51) Int. Cl.: A61K 9/20, A61K 31/164, A61K 47/10, A61K 47/32, A61K 45/00

(54) **TABLETS QUICKLY DISINTEGRATING IN ORAL CAVITY**
TABLETTEN, DIE IN DER MUNDHÖHLE SCHNELL ZERFALLEN
COMPRIMES A DELITEMENT RAPIDE DANS LA BOUCHE

(30) Priority: 06.03.2001 JP 2001062678
(43) Date of publication of application: 03.12.2003
(73) Proprietor: Kyowa Hakko Kirin Co., Ltd., Tokyo 100-8185 (JP)
(72) Inventor: OHTA, Motohiro, c/o Fuji Plant, Kyowa Hakko, Sunto-gun, Shizuoka 411-8731 (JP); KUBOYAMA, Muneko, c/o Fuji Plant, Kyowa Hakko, Sunto-gun, Shizuoka 411-8731 (JP); YOSHIMOTO, Hirokazu, c/o Fuji Plant, Kyowa Hakko, Sunto-gun, Shizuoka 411-8731 (JP); WATANABE, Yasushi, c/o Fuji Plant, Kyowa Hakko, Sunto-gun, Shizuoka 411-8731 (JP); MORIMOTO, Kiyoshi, c/o Fuji Plant, Kyowa Hakko, Sunto-gun, Shizuoka 411-8731 (JP); KOSUGI, Makoto, c/o Taisho Pharmaceutical Co. Ltd., Tokyo 170-8633 (JP)
(74) Representative: Teipel, Stephan
(86) International application number: PCT/JP2002/002048
(87) International publication number: WO 2002/069933

(56) References cited:
- EP-A- 0 914 818
- WO-A-00/09095
- WO-A-00/57857
- WO-A-00/78292
- WO-A1-00/78292
- JP-A- 10 298 061
- JP-A- 11 012 162
- JP-A- 61 085 330
- JP-A- 2000 273 039
- US-A- 5 573 777
- US-A- 6 106 861

## Description

### TECHNICAL FIELD

The present invention relates to an intraorally rapidly disintegrable tablet.

### BACKGROUND ART

As for the formulation of medicine for oral administration, there may be principally exemplified solid preparations such as granules, powders, fine particles, tablets or capsules, or liquid preparations such as syrups. Among the above, granules, powders or fine particles often give people who take them an unpleasant feeling, because they give rough feeling to the tongue or they are stuck between teeth. Solid preparations including tablets and capsules are easier to treat for the recipients as compared with granules, powders or fine particles, and have been widely used as medicines for oral administration. However, these solid preparations are difficult to be taken by an aged person or child who has a weak swallowing strength due to deglutition difficulties through the gullet. Such solid preparations are aimed for their medicinal ingredients to be disintegrated, dissolved and absorbed in the digestive tract, and thus rapid disintegrability or dissolvability is not generally exerted in the oral cavity. While syrup is a formulation which can be easily taken even by the aged or children, it has a problem of being inconvenient in measuring an accurate dose.

In view of the present situation of medicinal preparations as mentioned above, there has been expected, considering the aging society and the changes in people's living environment, to develop an intraorally disintegrable solid preparation which not only preserves the characteristics of tablets, i.e., convenience in treatment, but also can be taken easily even by a patient like an aged person or a child who has a weak swallowing strength, and further can be taken without water at any time and at any place. The tablet, if it is intraorally rapidly disintegrable, may be easily taken even by a patient who has a weak swallowing power, and it can be administered at an exact dose unlike liquid preparations such as syrup and so on. Moreover, considering that patients in general other than said patients can also take the tablet without water in the outdoors where water is not ready, an intraorally disintegrable solid preparation is a formulation which can improve taking compliance for all patients.

A tablet with rapid disintegration only can be easily obtained by compression-molding a medicinal ingredient prescribed at low pressure. However such a tablet has a low hardness and it is likely that the shape is hardly maintained and crushed in the course of packaging or distribution or when the recipient pushes the tablet out of the package to take it. Accordingly, an intraorally rapidly disintegrable tablet should have not only an excellent intraoral disintegrability, but also the hardness which is sufficient to present no problem in handling. However the solubility of a tablet is generally antinomic to the hardness of the tablet. Namely, improving the solubility of a tablet to increase the speed of dissolution results in deterioration in its hardness. Nevertheless the hardness of the tablet is a principal element in the course of packaging or distribution or when the recipient takes the tablet out of the package for intake as set forth hereinabove. If the hardness of the tablet is insufficient, the tablet cannot preserve its shape and might be crushed up in said course and process.

As for a technique for producing a tablet which is disintegrated and dissolved rapidly in the oral cavity, there has been proposed a method wherein the medicinal ingredients employed conventionally are dissolved or suspended in an aqueous medium, filled in a pocket of a blister pack which is previously molded, and solution is subjected to freeze drying or vacuum drying to be dehydrated. These solid preparations have rapid solubility, however, they have such problems that sufficient mechanical strength cannot be obtained or it is difficult to treat in a usual manner due to their high hygroscopicity. In addition, since the manufacture of such solid preparations is carried out by the method wherein the various kinds of components are dissolved while heating, molded by filling, and solidified through cooling, or by the method wherein the various kinds of components in a wet condition are molded by filling or under pressure, and dried, there is a problem of taking a lot of time, and so on.

Meanwhile researches on the manufacture of preparations disintegratable in the oral cavity by the wet molding method, which has mostly been used for producing tablets, have also been pursued. Japanese Unexamined Patent Publication No. 48726/1997 discloses a rapidly disintegrating preparation in a mouth cavity, which comprises a medicine together with materials to preserve the shape of the formulation, the formulation is produced by humidified ingredients to mold in the wet condition or by chemical agents and subsequently dried. As the materials to preserve the shape of the formulation, sugars, sugar alcohols and high molecular compounds soluble in water are exemplified. Japanese Unexamined Patent Publication No. 271054/1993 discloses a process for tableting a mixture of medicine, sugars and water to get the sugar particles with a wet surface to then be dehydrated, and Japanese Unexamined Patent Publication No. 291051/1996 discloses a process wherein a mixture of a medicine a water-soluble binding agent and a water-soluble excipient is tableted under low pressure, which is subsequently humidified and finally dehydrated.

However, in those methods for production, a moisturized mixture is tableted, and conventional tableting machines are difficult to be used. Further, in the methods, the tablet should be humidified and dehydrated after subjected to tableting, which results in numerous steps in production. Moreover, the methods raise a serious problem that they cannot be applied to medicines unstable in water.

Japanese Patent Publication No. 502194/1994 (US patent No. 5,464,632) discloses a rapidly disintegratable multiparticular tablet, comprising an active substance in the form of coated microcrystals or uncoated microgranules and a mixture of excipients, which disintegrates in a short time of less than 60 seconds in the mouth. Said patent discloses that a disintegrating agent is contained, however, the disintegrating agent is not specifically defined therein. Moreover, said patent does not disclose at all that D-mannitol is comprised as an excipient.

Furthermore, Japanese Unexamined Patent Publication No. 35450/1999 discloses an improved multiparticular tablet, which disintegrates in less than 40 seconds in the mouth, and comprises an active ingredient in the form of coated microcrystals and an excipient. The tablet contains, as the excipient, at least one decomposer such as crospovidone and polyhydric alcohol such as mannitol in the form of a product directly compressible and having an average particle diameter of 100 to 500 µm or in the form of particles having an average particle diameter of less than 100 µm. However, the reference is quite silent on the specific surface area of mannitol.

On the other hand, D-mannitol is excellent in stability and mixability with a physiologically active substance, and shows no humidification and does not retain moisture, and thus is an excipient valuable for use for a tablet or capsule containing a physiologically active substance, which is greatly sensitive to water. However D-mannitol has a weak binding property in compression molding and further causes a great friction to a metal wall face. Therefore when included as an excipient, mannitol causes die friction or capping in the process of compression molding, and so cannot give the adequate hardness for the tablets. Mannitol also causes friction to the die wall face and die side face and causes sometimes trouble in operating the tableting machine. Accordingly the use of D-mannitol as an excipient has been practically restricted to extremely limited formulations such as for tablets of mastication.

Meanwhile, D-mannitol is a crystalline powder classified according to X-ray diffraction pattern into α, β and δ type [Walter-Levy, L., Acad. Sci. Parist. 276 Series C. 1779, (1968)]. It has been generally known in respect to the improvement on the moldability of crystalline powder that the number of binding points increases by crushing crystals finely and thus moldability (the binding property in the process of compression molding) is improved. However, crushing finely D-mannitol not only merely promotes the friction to the metal wall face in the process of compression molding but also raises an issue of the fluidity and deterioration in terms of handling. Accordingly it has been a rare case that mannitol is used independently when used as an excipient for a compression molding product. Mannitol is rather mixed with other excipients having good moldability, e.g., sugars and also mixed with other additive such as binding agents, fillers, etc. for the use.

In regard to the method for improving the moldability of D-mannitol, Japanese Unexamined Patent Publication No. 85330/1986 discloses a method for producing an excipient for direct tableting, which is characterized by spray drying mannitol, and Japanese Unexamined Patent Publication No. 36291/1998 discloses a solid composition comprising D-mannitol with a specific surface area of at least about 1 m²/g as an excipient having excellent compression moldability. However the processes for producing mannitol used in said inventions are complicated and expensive. Further, during the production of the tablets, the fluidity of the particle is not exerted sufficiently and the mixability with an active ingredient is not adequate. Thus, it is understood that the uniformity of the tablet weight and content is deteriorated.

WO97/47287 discloses a tablet comprising sugar alcohol including D-mannitol, which has an average particle diameter of less than 30 µm. However, there has been left room for further improvement to shorten the intraorally disintegration time, when a water-soluble medicine is administered.

EP 1 203 580 A1 discloses quickly disintegrating solid preparations which contain an active ingredient, D-mannitol having an average particle size of 30 µm to 300 µm, a disintegrating agent and cellulose.

### DISCLOSURE OF THE INVENTION

An object of the present invention is to provide an intraorally rapidly disintegrable tablet, which disintegrates rapidly without water in the oral cavity and has a practical hardness so as not to cause such problem as losing its shape in the process of production and distribution as well as while being handled in a hospital or handled by patients.

In order to accomplish the above object, the present inventors have conducted investigations by trial and error firstly on excipients with respect to an intraorally rapidly disintegrable tablet, which comprise as the principal medicine a water-soluble pharmacologically active ingredient with a water solubility of preferably at least 0.5 mg/ml or more, preferably at least 1 mg/ml, and as a result, they found out unexpectedly that an intraorally rapidly disintegrable tablet which has an excellent disintegrability and a practical hardness can be obtained by including D-mannitol in the form of crystals or fine particles with primary particle average diameter of about 30 pm or more and a specific surface area of about 0.4 m²/g or less. The inventors also conducted investigations by trial and error on disintegrating agents and have found out that crospovidone can be desirably used, and the intraorally disintegration time is lengthened when the disintegrating agents other than crospovidone are used.

The inventors have endeavored to provide a novel method for producing the intraorally rapidly disintegrable tablets, and have found that an intraorally rapidly disintegrable tablet having a hardness causing no problem in a practical use, i.e. about 20 N or more, and disintegrating rapidly in the oral cavity within about 30 seconds can be obtained easily through a process, wherein an aqueous medium is added to a powdery particle comprising a pharmacologically active ingredient, D-mannitol in the form of crystals or fine particles, and crospovidone, the mixture is kneaded and granulated, the resulting granules are dried, other additives are optionally added to the granules to prepare a material for compression molding, and the material is subjected to compression molding. Particularly the inventors have also found an astonishing fact that the disintegration time can be further shortened (within about 20 seconds) according to a method wherein the material for compression molding containing no lubricant is molded by a compression molding machine, in which a lubricant is previously applied to the punch surface and to the die wall face (the tableting process with external lubricating).

As such, the inventors studied further and steadily to accomplish the present invention.

Thus, the present invention relates to the following.
(1) An intraorally rapidly disintegrable tablet according to claims 1-9.
(2) The tablet according to the above (1), wherein the pharmacologically active ingredient is midodrine hydrochloride.
(3) The method for producing an intraorally rapidly disintegrable tablet according to claims 10-19.
(4) The method for producing a tablet according to the above (3), wherein the pharmacologically active ingredient is midodrine hydrochloride.

Here, the pharmacologically active ingredient in the above (1) and (3) is preferably a pharmacologically active ingredient with a water solubility of at least 1 mg/ml.

### BEST MODE OF CARRYING OUT THE INVENTION

The pharmacologically active ingredient in the tablet of the present invention is not particularly limited, so long as it has a physiological activity such as a pharmacological activity and is designed for oral administration. However, the pharmacologically active ingredients have a water solubility of at least 0.5 mg/ml, preferably at least 1 mg/ml. The solubility of the pharmacologically active ingredients can be determined by the method described in the Japanese Pharmacopoeia; General Rule 13; Paragraph 23, more specifically the determination is carried out by measuring the amount of powder dissolved in water within 30 minutes when stirred strongly at 20±5 °C for 30 seconds in every 5 minutes.

Specific pharmacologically active ingredients include, for example, one or more ingredient (s) selected from the group consisting of antipyretic analgesic antiphlogistics, psychotropic agents, anxiolytics, anti-depressants, central nervous system acting drugs, digestives, antiulcer agents, antacids, hypnotic sedatives, cerebral metabolism improving agents, antitussive expectorants, antiallergic agents, bronchodilators, cardiacs, antiarrhythmic agents, antihistamines, diuretics, hypotensive drugs, vasoconstrictors, cholagogues, antihyperlipemic agents, coronary vasodilator drugs, peripheral vasodilator drugs, antibiotics, remedies for Alzheimer's disease and gout treating agents.

The "Pharmacologically active ingredients with a water solubility of at least 0.5 mg/ml" include, for instance, antipyretic analgesic antiphlogistics such as acetaminophen and dichlorofenac sodium, hypnotic sedatives such as flurazepam hydrochloride, antiepileptic agents such as fenitoin sodium, curatives for Parkinson's disease such as amadazine hydrochloride, psychotropic agents such as imipran hydrochloride and clocapramine hydrochloride, cardiacs such as aminopyrine and ethylefrin hydrochloride, antiarrhythmic agents such as oxprenolol hydrochloride and mexiletine hydrochloride, hypotensive drugs such as captopril and hydralazine hydrochloride, vasoconstrictors such as midodorine hydrochloride and amezinium metilsulfate, vasodilator drugs such as dilazep hydrochloride and diltiazem hydrochloride, antitussives such as pentoxverine citrare, expectorants such as ambroxol hydrochloride and methyl L-cystein hydrochloride, bronchodilators such as clenbuterol hydrochloride and procaterolhydrochloride, and antiulcer agents such as cetraxate hydrochloride, pirenzepine hydrochloride and famotidine. The water solubility of midodrine hydrochloride, captopril, ambroxol hydrochloride, loperamide hydrochloride and famotidine are, for example, about 100 mg/ml, about 60 mg/ml, about 20 mg/ml, about 1 mg/ml, and about 0.7 mg/ml, respectively.

The pharmacologically active ingredient in the intraorally rapidly disintegrable tablet of the present invention may be a pharmacologically acceptable salt of the compound having the physiological activity and the like. Examples of the pharmacologically acceptable salt include salts with an inorganic acid (e.g. , hydrochloric acid, sulfuric acid, nitric acid, etc.), an organic acid (e.g., carbonic acid, bicarbonic acid, succinic acid, acetic acid, propionic acid, trifluoroacetic acid, etc.), an inorganic base (e.g., alkali metal such as sodium or potassium, alkaline earth metal such as calcium or magnesium, etc.) or an organo-basic compound (e.g., organo-amines such as triethylamine and the like, basic amino acids such as arginine and the like, etc.).

Accordingly, in the case where the pharmacologically active ingredient in the intraorally rapidly disintegrable tablet of the present invention is midodrine, the pharmacologically acceptable salt of midodorine, specifically such as midodrine hydrochloride, may be included.

The primary particle of D-mannitol in the intraorally rapidly disintegrable tablet of the present invention is characterized in that it is in the form of crystals or fine particles with primary particle average diameter of at least 30 µm and a specific surface area of 0.4 m²/g or less. Here, the primary particle does not indicate a granulated lump formed by cohering particles but means an individual particle composing the granulated lump.

The desirable embodiments of the crystals or fine particles are crystals with primary particle average diameter of from 30 to 1000 µm, preferably from 35 to 300 µm, more preferable, from 40 to 100 µm, and a specific surface area of from 0.4 to 4x10⁻²⁰ m²/g, preferably from 0.3 to 2x10⁻⁶ m²/g, more preferably from 0.3 to 0.02 m²/g.

The average particle diametermeans 50 % particle diameter, which indicates the particle diameter manifested at the point of 50 % in the graph of cumulative percentage. Determination of the particle diameter can be carried out for instance by the method of laser diffraction for analyzing particle size distribution, more particularly by using a Laser Scattering Particle Size Distribution Analyzer LA-910 (produced by HORIBA Ltd.) for measuring the particle size distribution. The specific surface area can be determined by the BET (Brunauer-Emmert and Teller's) method, specifically by using the measuring instrument SA-9603 manufactured by Horiba Ltd., for instance.

D-mannitol used in the present invention may be in any crystal form of type α, type β and type δ. The D-mannltol crystal of type α, type β and type δ are defined according to the classifications of the polymorphic crystals of D-mannitol by the X-ray diffraction pattern reported by Walter-Levy, L. et al (Acad. Sci. Parist. 276 Series C, 1779, (1968)). The D-mannitol may be also the one obtained by a known method per se such as liquid extraction from seaweeds, ammonia electrolytic reduction of glucose solution, contact reduction of sucrose solution and so on.

As long as the D-mannitol crystals in the intraorally rapidly disintegrable tablet of the present invention have the above features, the preparation method thereof is not limited. For example, mentioned is a method in which the D-mannitol particle is crushed into a desired size by a known grinding mill such as a jet mill, a hammer mill, a ball mill, a vibration ball mill or a pin mill. Alternatively, D-mannitol in the form of desired crystals can also be obtained by dissolving D-mannitol in an adequate solvent or desirably in water (it may be heated up to from 60 to 80 °C) to give a solution of preferably from 10 to 40 % by weight, and subjecting the solution to spray-drying with a spray drier which is publicly known. The condition of the spray drying is not particularly limited, but the exhaust heat temperature is preferably from 120 to 140°C.

As a desirable embodiment for manufacturing D-mannitol in the form of crystals used in the present invention, it may be exemplified a method for obtaining D-mannitol in the form of crystals with a specific surface area of 0.4 m²/g or less which comprising treating D-mannitol such as spray drying without increasing the specific surface area. Further, if the crystals obtained have primary particle average diameter of at least 30 µm, they may be optionally crushed by a grinding mill such as a hammer mill or a jet mill. That is, the crystals having a desired particle diameter, provided that the primary particle average diameter is at least 30 µm, can be obtained either by adjusting the screen diameter in case a hammer mill is used or by controlling the condition of air volume and particle-supplying speed in case a jet mill is used.

As crospovidone contained in the intraorally rapidly disintegrable tablet of the present invention, any cross-linking polymerization products from 1-vlnyl-2-pyrrolidone can be used, and usually crospovidone having a molecular weight of more than 1,000,000 is employed. Having been widely known as a disintegrating agent, the crospovidone can be produced by a known method *per se.* Alternatively, the commercially available product including Collidone CL (made by BASF Japan), Polyplasdone XL (made by ISP in Japan) may be used.

In regard to the intraorally rapidly disintegrable tablet of the present invention, the proportion of the pharmacologically active ingredient (the compounding ratio by weight of the pharmacologically active ingredient relative to the total weight of the tablet) is not particularly limited, however, it is preferably from 0.01 to 50 % bey weight, more preferably from 0.01 to 30 % by weight.

In regard to the intraorally rapidly disintegrable tablet of the present invention, the proportion of the D-mannitol (the compounding ratio by weight of D-mannitol relative to the total weight of the tablet) is preferably from 20 to 99 % by weight.

In regard to the intraorally rapidly disintegrable tablet of the present invention, the proportion of crospovidone (the compounding ratio by weight of crospovidone relative to the total weight of the tablet) is not particularly limited, however, it is preferably from 0.5 to 30 % by weight, more preferably from 0.5 to 20 % by weight, most preferably from 1 to 10.% by weight.

In regard to the intraorally rapidly disintegrable tablet of the present invention the proportion (compounding ratio) of the pharmacologically active ingredient, D-mannitol in the form of crystals or fine particles and crospovidone can be appropriately determined according to the physical characteristics such as solubility, particle size and wettability of the pharmacologically active ingredient.

A lubricant is included in the intraorally rapidly disintegrable tablet of the present invention. The lubricant is not particularly limited, and preferably used are, for example, magnesium stearate, calcium stearate, stearic acid, stearyl alcohol, sodium stearyl fumarate, talc and sucrose fatty acid ester.

The compounding ratio by weight of the lubricant relative to the intraorally rapidly disintegrable tablet of the present invention is preferably from 0.001 to 2 % by weight, more preferably from 0.01 to 1 % by weight, most preferably from 0.05 to 0.5 % by weight.

The lubricant is included only on the surface of the tablet by the external lubrication tableting method described below.

The intraorally rapidly disintegrable tablet of the present invention may comprise one or more additive component (s) selected from the group consisting of a flavor, a coloring agent, a corrigent, a sweetener, a stabilizer, an antioxidant, a fluidizing agent and an auxiliary solubilizer. The compounding rate by weight of the additives relative to the total weight of the tablet is preferably less than 5% by weight, more preferably less than 1 % by weight, most preferably less than 0.5 % by weight.

Examples of the flavor include L-menthol, lemon, orange, strawberry and mint.

Examples of the coloring agent include yellow iron sesquioxide, red iron sesquioxide, food colors such as Yellow No. 5 and Blue No. 2, and food lake colors.

Examples of the corrigent and sweetener include aspartame, sodium saacharate, stevia, dipotassium glycyrrhizate.

Examples of the fluidizing agent include light anhydride silicic acid, calcium silicate and synthetic aluminum silicate.

Examples of the stabilizing agent include benzoic acid, sodium benzate and citric acid.

Examples of the antioxidant include ascorbic acid and tocopherol.

Examples of the auxiliary solubilizer include principally a surfactant, more particularly such as polysorbate 80, sodium laurylsulfate or macrogol 400.

The additives added in the intraorally rapidly disintegrable tablet of the present invention are not particularly limited. It is preferable that a saccharide and a binding agent other than D-mannitol, which exert high viscosity when contacted with water, are not contained in the tablet in the present invention. Thus, the disintegrability of the intraorally rapidly disintegrable tablet can be improved by excluding the other saccharide and binding agent mentioned below.

The saccharides other than D-mannitol include, for instance, saccharides such as white sugar, glucose, galactose and fructose, sugar alcohols such as sorbitol and maltitol. Binding agents other than D-mannitol include, for instance, hydroxypropylcellulose (HPC), hydroxymethylpropylcellulose (HPMC), methylcellulose (MC), polyvinylpyrrolidone (PVP), pluran, polyvinylalcohol (PVA) and gelatin.

Such intraorally rapidly disintegrable tablet of the present invention comprising (a) a pharmacologically active ingredient with a water solubility of at least 0.5 mg/ml, preferably at least 1 mg/ml as a principal component, (b) D-mannitol in the form of crystals with primary particle average diameter of at least 30 µm and a specific surface area of 0.4 m²/g or less, (c) a crospovidone, (d) a lubricant and (e) optionally other additives, has a practical hardness and disintegrates rapidly in the oral cavity without water.

The "practical hardness of the tablet" varies depending on the components and shape of the tablet, however, the appropriate hardness is preferably 20N or more, more preferably 30N or more. The hardness of the tablet can be determined by a conventional hardness meter, e.g., the hardness meter TH-303 (produced by Toyama Chemical Co., Lid.).

"Rapid disintegration" means disintegration by the action of saliva without water in the oral cavity within a desirable time without causing a practical problem. While the "disintegration time without causing a practical problem" varies depending on individuals, it is preferably not longer than 30 seconds. The disintegration time can be determined by measuring the time within which the intraorally disintegrable tablets are completely disintegrated by the action of saliva in the oral cavity of healthy male and female adults.

The intraorally rapidly disintegrable tablet of the present invention is not limited to those which are taken without water and has a merit in that even a patient with little saliva can easily take the tablet when taking it with water. Accordingly it can be said that the tablet of the present invention is much easier to take compared to conventional tablets. The intraorally rapidly disintegrable tablet of the present invention can be taken by, for example, (a) keeping the tablet in the mouth without swallowing and taking it after it is disintegrated or dissolved with small quantitiy of water or saliva in the oral cavity without water, (b) swallowing the tablet as it is with water, and (c) taking the tablet after it is dissolved or disintegrated in water.

The intraorally rapidly disintegrable tablet of the present invention can be administered orally without water as described above, and therefore the tablet is particularly useful in cases where (a) the tablet should mostly be taken without water, (b) the patient has difficulty in swallowing a tablet or (c) the patient is an aged or infant patient who may get conventional tablets stuck in the throat. Preferred examples of the drug for the case (a) include drugs such as antipyretics, anodynes, antiphlogistics, anxiolytics, antitussive expectorants, antivertigo agents, and prophylactic remedies for motion sickness. Preferred examples for the case (b) include preventive and therapeutic medicines for hypertension, hyperlipidemia, diabetes, bronchial asthma and cerebrovascular accident.

The intraorally rapidly disintegrable tablet of the present invention can be produced by conventional methods employed in the pharmaceutical field, i.e. , direct or indirect tableting methods.
For example, mentioned is a method wherein the pharmacologically active ingredient, the D-mannitol, the crospovidone, together with other additives as desired are mixed and the mixture is subjected to compression molding.

The mixing is conducted by devices such as a vertical granulator VG 10 (produced by POWREX Corporation), all-purpose blending machine, fluidized bed granulator, V type mixer and tumbler mixer. Compression molding is carried out by conventional tableting machines such as single shot tableting machine or rotary type tableting machine.

As the preferable embodiment of the method for the production of the intraorally rapidly disintegrable tablet of the present invention, mentioned is a method in which an aqueous medium is added to a powdery particle comprising the pharmacologically active ingredient, the D-mannitol in the form of crystals, and the crospovidone; the mixture is kneaded and granulated; the resulting granules are dried; other additives are optionally added to the granules to prepare a material for compression molding; and the material is subjected to compression molding.

In the above method, examples of the aqueous medium include purifiedwater, methanol, ethanol, acetone, and mixtures thereof, and the mixing ratio can be appropriately determined. Among them, purified water is preferably used.

The amount of the aqueous medium added to the powdery particle is preferably 5 to 30 % by weight.

The method for granulating the powdery particle comprising the pharmacologically active ingredient, D-mannitol, and crospovidone is not particularly limited, and dry granulation is employable, however, preferable is the method in which all or a part of the components is prepared through wet granulation such as fluidized bed granulation, tumbling fluidized bed mixing granulation, mixing granulation (desirably by the high speed mixing granulator, the type of NSK-250 produced by OKADA SEIKO company or the high speed mixing granulator VG-25 produced by POWREX Corporation) and extruding granulation (desirably by a granulat DGL-1 produced by FUJI PAUDAL, Japan). The particle size and the specific surface area of the powdery particle can be optionally determined as long as the mixture has a fluidity not causing any problem in the granulation.

Other additives e.g. a flavor, a stabilizer and the like, may be added in the powdery particle to be mixed, but it is rather desirable to mix the additive with the granulated material obtained by the above process, as described below.

Subsequently the granulated material is dehydrated to give a granule. The dehydration method is not specifically limited and the dehydration is preferably carried out by using a conventional drier, for instance, either by a ventilation box type drier (preferably the Japanese drier of TE-98 type) at 40 to 80 °C for 30 to 90 minutes or by a fluidized bed drier (preferably the drier of FLO-5 type made by the FREUND Industrial Co., Ltd.) at 70 to 90°C for 5 to 30 minutes.

Alternatively, the granulation and dehydration can be carried out all at once by a fluidized bed granulating drier (preferably the drier of FLO-2 type made by the FREUND Industrial Co., Ltd.).

To the obtained granules are added the aforementioned additives other than the lubricant, such as a flavor and a stabilizing agent, to prepare the materials for compression molding. Subsequently, the materials are subjected to compression molding to give the intraorally rapidly disintegrable tablets of the present invention. Compression molding can be carried out by conventional tableting machines, preferably by a tablet machine with excellent productivity, such as a single shot tableting machine with excellent productivity, a rotary type tableting machine or a hydraulic compressor. The pressure at compression molding is 3 to 30 kN, preferably 5 to 20 kN.

As other preferable embodiments of the method for production of the intraorally rapidly disintegrable tablet of the present invention, mentioned is a method wherein the pharmacologically active ingredient, D-mannitol, crospovidone, and optionally the aforementioned additive are mixed by a conventional mixer, such as a V type mixer or a tumbler mixer and then the resulted mixture is directly subjected to tableting.

In the method for production of the intraorally rapidly disintegrable tablet of the present invention, the lubricant is previously applied to the punch surface and to the die wall face of the compression-molding machine without being mixed with the other components, and then the materials for compression molding are subjected to compression molding (external lubrication tableting method). The tablet produced in this way, has advantages in that it has higher hardness, and it disintegrates in short time. The method for applying the lubricant to the punch surface and to the die wall face is not particularly limited.

The weight and shape of the intraorally rapidly disintegrable tablet of the present invention are not specially limited. For example, a round shape or various kinds of shapes having a surface such as a regular round surface, sugar-coated round surface, flat with a square shaped corner, flat with a round shaped corner or double layered round surface can be adopted as the shape of the tablet formulation. The preparation may also be used as a dividable tablet having cleavage line(s). It may further be a multi-layered tablet having two or more layers.

Above all, it is preferable that the intraorally rapidly disintegrable tablet of the present invention is a small one. The hardness and the intraoral disintegration time of the tablet can be also controlled by its weight and/or shape. As the tablet of the present invention, for instance, a tablet with the weight of 80 to 250 mg and having tablet diameter of 6 to 9 mm is preferable.

The intraorally rapidly disintegrable tablet of the present invention can be safely administered orally to human and mammalians other than human, e.g., rats, mice, rabbits, cats, dogs, cattle, horses and monkeys. The dose of the intraorally rapidly disintegrable tablet of the present invention varies depending on the pharmaceutical agents, the subject of administration and types of diseases, and it can be appropriately determined from the range of dose that is an effective amount as a medical component. The intraorally rapidly disintegrable tablet of the present invention can be administered once or several times, preferably two to four times a day.

### EXAMPLES

The present invention will be further illustrated in the following Examples and Comparative Examples, but those examples are not intended to limit the scope of the invention.

The water solubility of the pharmacologically active ingredient was determined by the method described in the Japanese Pharmacopoeia, the General Rule 13, Paragraph 23. Primary particle average diameter (hereinafter simply referred to as "average particle diameter") of D-mannitol means the particle diameter at the point of 50 % when determined by a Laser Scattering Particle Size Distribution Analyzer LA-910 (produced by HORIBA Ltd.). The specific surface area of D-mannitol was determined by instrument SA-9603 (produced by HORIBA Ltd.) according to the BET (Brunauer-Emmert and Teller's) method.

### Example 1

20 g of midodrine hydrochloride (water solubility: about 100 mg/ml), 1120 g of D-mannitol (produced by KYOWA HAKKO KOGYO Co., Ltd./Nikken Chemical and Synthetic Industry Co., Ltd.; average particle diameter: 60 µm, specific surface area: 0.1 m²/g) and 60 g of crospovidone ("POLYPLASDON XL-10", produced by ISP) were introduced into a high speed mixing granulator (NSK-250 type, produced by OKADA SEIKO Co., Ltd.). The mixture was kneaded for five minutes after adding 200 g of purified water. The kneaded mixture was granulated by an extruding granulator (DGL-1 type, produced by FUJI PAUDAL Co., Ltd.) installed with a 0.8 mm Ø screen to prepare granules. The granules were dried by a ventilation box type drier (the Japanese drier TE-98) at 60°C for 60 minutes. The dried granules were passed through a No. 20 wire gauze to give even granules for tableting. Subsequently, the granules were subjected to compression molding by a rotary type tableting machine equipped with punches having a 7.0 mm ∅ flat face and equipped with a device for applying calcium stearate to the punch surface and to die wall face prior to filling the granules in the die of the tableting machine to produce tablets having a weight of 120 mg and the thickness of 2.6 mm. The tableting pressure was about 7.5 kN. About 0.1% by weight of calcium stearate (lubricant) was adhered to the surface of the tablet obtained.

### Example 2

20 g of midodrine hydrochloride (water solubility: about 100 mg/ml), 1156 g of D-mannitol as used in Example 1 and 24 g of crospovidone ("POLYPLASDON XL-10", produced by ISP) were introduced into a high speed mixing granulator (NSK-250 type, produced by OKADA SEIKO Co., Ltd.). The mixture was kneaded for five minutes after adding 200 g of purified water. The kneaded mixture was granulated by an extruding granulator (DGL-1 type, produced by FUJI PAUDAL Co., Ltd.) installed with a 0.8 mm ∅ screen to prepare granules. The granules were dried by a ventilation box type drier (the Japanese drier of TE-98 type) at 60°C for 60 minutes. The dried granules were passed through a No. 20 wire gauze to give even granules for tableting. Subsequently, the granules were subjected to compression molding by a rotary type tableting machine equipped with punches having a 7.0 mm ∅ flat face and equipped with a device for applying calcium stearate to the punch surface and to the die wall face prior to filling the granules in the die of the tableting machine to produce tablets having a weight of 120 mg and thickness of 2.6 mm. The tableting pressure was about 7.5 kN. About 0.1% by weight of calcium stearate (lubricant) was adhered to the surface of the tablet obtained.

### Example 3

20 g of midodrine hydrochloride (water solubility: about 100 mg/ml), 1120 g of D-mannitol (produced by KYOWA HAKKO KOGYO Co., Ltd./Nikken Chemical and Synthetic Industry Co., Ltd., average particle diameter: 35 µm, specific surface area: 0.3 m²/g) and 60 g of crospovidone ("POLYPLASDON XL-10", produced by ISP) were introduced into a high speed mixing granulator (NSK-250 type, produced by OKADA SEIKO Co., Ltd.). The mixture was kneaded for five minutes after adding 200 g of purified water. The kneaded mixture was granulated by a extruding granulator (DGL-1 type, produced by FUJI PAUDAL Co., Ltd.) installed with a 0.8 mm ∅ screen and dried by a ventilation box type drier (the Japanese drier of TE-98 type) at 60°C for 60 minutes. The dried granules were passed through a No. 20 wire gauze to give even granules for tableting. Subsequently, the granules were subjected to compression molding by a rotary type tableting machine equipped with punches having a 7.0 mm Ø flat face and equipped with a device for applying calcium stearate to the punch surface and to the die wall face prior to filling the granules in the die of the tableting machine to produce tablets having a weight of 120 mg and thickness of 2.6 mm. The tableting pressure was about 7.5 kN. About 0.1 % by weight of calcium stearate (lubricant) was adhered to the surface of the tablet obtained.

### Example 4

20 g of midodrine hydrochloride (water solubility: about 100 mg/ml), 1060 g of D-mannitol as used in Example 1 and 120 g of crospovidone ("POLYPLASDON XL-10", produced by ISP) were introduced into a high speed mixing granulator (NSK-250 type, produced by OKADA SEIKO Co., Ltd.). The mixture was kneaded for five minutes after adding 200 g of purified water. The kneaded mixture was granulated by an extruding granulator (DGL-1 type, produced by FUJI PAUDAL Co., Ltd.) installed with a 0.8 mm ∅ screen to prepare granules. The granules were dried by a ventilation box type drier (the Japanese drier of TE-98 type) at 60°C for 60 minutes. The dried granules were passed through a No. 20 wire gauze to give the even granules for tableting. Subsequently, the granules were subjected to compression molding by a rotary type tableting machine equipped with punches having a 7.0 mm ∅ flat face and equipped with a device for applying calcium stearate to the punch surface and to the die wall face prior to filling the granules in the die of the tableting machine to produce tablets having a weight of 120 mg and a thickness of 2.6 mm. The tableting pressure was about 7.5 kN. About 0.1 % by weight of calcium stearate (lubricant) was adhered to the surface of the tablet obtained.

### Comparative Example 1

200 g of domperidone (water solubility: about 2 µg/ml), 2080 g of D-mannitol (produced by KYOWA HAKKO KOGYO Co., Ltd./Nikken Chemical and Synthetic Industry Co., Ltd.; specific surface area: 0.1 m²/g, average particle diameter: 60 µm) and 120 g of crospovidone were introduced into a fluidized bed granulating drier (FLO-2 type, produced by FREUND Industrial Co., Ltd.). The mixture was granulated by spraying 50 ml of purified water to prepare granules. 1190 g of the granules and 10 g of magnesium stearate were mixed to prepare mixture for tableting. Subsequently, the mixture was subjected to compression molding by a rotary type tableting machine installed with punches having a 7mm ∅ plane to produce tablets having a weight of 120 mg. The thicknesses of the tablets were each 2.55, 2.60 and 2.65 mm, and the tableting pressures were about 10, about 7.5 and about 6 kN, respectively. Thus obtained tablet contains 0.8 % by weight of magnesium stearate (lubricant).

### Comparative Example 2

2260 g of D-mannitol (produced by KYOWA HAKKO KOGYO Co., Ltd./Nikken Chemical and Synthetic Industry Co., Ltd.; specific surface area: 0.1 m²/g, average particle diameter: 60 µm) and 120 g of crospovidone were introduced into a fluidized bed granulating drier (FLO-2 type, produced by FREUND Industrial Co., Ltd.). The mixture was granulated by spraying 50ml of purified water to prepare granules. 1190 g of the granules and 10 g of magnesium stearate were mixed to prepare mixture for tableting. Subsequently, the mixture were subjected to compression molding by a rotary type tableting machine installed with punches having a 7mm ∅ plane to produce tablets having a weight of 120 mg. The thicknesses of the tablets were each 2.55, 2.60 and 2.65 mm, and the tableting pressures were about 10 kN, about 7.5 kN and about 6 kN, respectively. Thus obtained tablet contains about 1.0 % by weight of magnesium stearate (lubricant).

### Example 5

300 g of ambroxol hydrochloride (water solubility: about 20 mg/ml), 1980 g of D-mannitol as used in Example 1 and 120 g of crospovidone ("POLYPLASDON XL-10", produced by ISP) were introduced into a high speed mixing granulator (VG-25 type, produced by POWREX). The mixture was granulated by adding 400 g of purified water. The granules were dried by a fluidized bed drier (FLO-5 type, produced by FREUND Industrial Co., Ltd.) at 80 °C for 15 minutes. The dried granules were passed through a No. 20 wire gauze to give even granules for tableting. Subsequently, the granules were subjected to compression molding by a rotary type tableting machine equipped with punches having a 7.0 mm Ø flat face and equipped with a device for applying magnesium stearate to the punch surface and to the die wall face prior to filling the granules in the die of the tableting machine to produce tablets having a weight of 120 mg. The thicknesses of the tablets were 2.55, 2.60 and 2.65 mm, and the tableting pressures were about 10, about 7.5 and about 6 kN, respectively. About 0.1 % by weight of magnesium stearate (lubricant) was adhered to the surface of the tablet obtained.

### Example 6

300 g of ambroxol hydrochloride (water solubility: about 20 mg/ml), 1980 g of D-mannitol as used in Example 1 and 120 g of crospovidone ("POLYPLASDON XL-10", produced by ISP) were introduced into a high speed mixing granulator (VG-25 type, produced by POWREX). The mixture was granulated by adding 400 g of purified water. The granules were dried by a fluidized bed drier (FLO-5 type, produced by FREUND Industrial Co., Ltd.) at 80 °C for 15 minutes. The dried granules were passed through a No. 20 wire gauze to give even granules for tableting. 990 g of the granules was mixed with 10 g of magnesium stearate to prepare mixture for tableting. Subsequently, the granules were subjected to compression molding by a rotary type tableting machine installed with a punch having a 7.0 mm Ø flat face to produce tablets having a weight of 120 mg. The thicknesses of the tablets were each 2.55, 2.60 and 2.65 mm, and the tableting pressures were about 10, about 7.5 and about 6 kN, respectively. About 0.1 % by weight of magnesium stearate (lubricant) was adhered to the surface of the tablet obtained.

### Example 7

300 g of ambroxol hydrochloride (the water solubility: about_20 mg/ml), 1980 g of D-mannitol as used in Example 1 and 120 g of crospovidone ("POLYPLASDON XL-10", produced by ISP) were introduced into a high speed mixing granulator (VG-25 type, produced by POWREX). The mixture was granulated by adding 400 g of purified water. The granules were dried by a fluidized bed drier (FLO-5 type, produced by FREUND Industrial Co., Ltd.) at 80°C for 15 minutes. The dried granules were passed through a No. 20 wire gauze to give even granules. 990 g of the granules was mixed with 9 g of magnesium stearate to prepare mixture for tableting. Subsequently the mixture was subjected to compression molding by a rotary type tableting machine equipped with punches having a 7.0 mm Ø flat face and equipped with a device for applying calcium stearate to the punch surface and to the die wall face prior to filling the granules in the die of the tableting machine to produce tablets having a weight of 120 mg. The thicknesses of the tablets were each 2.55, 2.60 and 2.65 mm, and the tableting pressures were about 10, about 7.5 and about 6 kN, respectively. About 0.1 % by weight of magnesium stearate (lubricant) was adhered to the surface of the tablet obtained. In addition, about 0.9 % by weight of magnesium stearate (lubricant) is contained inside of the tablet.

### Example 8

15 g of loperamide hydrochloride (the water solubility: about 1 mg/ml), 1410 g of D-mannitol as used in Example 1 and 75 g of crospovidone ("POLYPLASDON XL-10" , produced by ISP) were introduced into a fluidized bed granulating drier (FLO-2 type, produced by FREUND Industrial Co., Ltd.). The mixture was granulated by spraying 50ml of purified water to obtain granules and the granules were dried. The dried granules were passed through a No. 20 wire gauze to give even granules. Subsequently, the granules were subjected to compression molding by a rotary type tableting machine equipped with punches having a 6.5 mm Ø flat face and equipped with a device for applying calcium stearate to the punch surface and to the die wall face prior to filling the granules in the die of the tableting machine to produce tablets having a weight of 100 mg and thickness of 2.3 mm. The tableting pressure was about 6 kN. About 0.1 % by weight of calcium stearate (lubricant) was adhered to the surface of the tablet obtained.

### Comparative Example 3

15 g of loperamide hydrochloride (water solubility: about 1 mg/ml), 1410 g of sorbitol (produced by TOWA chemical synthetic company, Japan) and 75 g of crospovidone ("POLYPLASDON XL-10", produced by ISP) were introduced into a fluidized bed granulating drier (FLO-2 type, produced by FREUND Industrial Co., Ltd.). The mixture was granulated by adding 50ml of purified water to obtain granules. The dried granules were passed through a No. 20 wire gauze to give even granules. Subsequently, the granules were subjected to compression molding by a rotary type tableting machine equipped with punches having a 6.5 mm Ø flat face and equipped with a device for applying calcium stearate to the punch surface and to the die wall face prior to filing the granules in the die of the tableting machine to produce tablets having a weight of 100 mg and thickness of 2.3 mm. The tableting pressure was about 6 kN. About 0.1 % by weight of calcium stearate (lubricant) was adhered to the surface of the tablet obtained.

### Comparative Example 4

15 g of loperamide hydrochloride (water solubility: about 1 mg/ml), 1410 g of white sugar (produced by NITSHIN sugar manufacturing company, Japan) and 75 g of crospovidone ("POLYPLASDON XL-10", produced by ISP) were introduced into a fluidized bed granulating drier (FLO-2 type, produced by FREUND Industrial Co., Ltd.). The mixture was granulated by adding 800 g of purified water to obtain granules and the granules were dried. The dried granules were passed through a No. 20 wire gauze to give even granules. Subsequently, the granules were subjected to compression molding by a rotary type tableting machine equipped with punches having a 6.5 mm ∅ flat face and equipped with a device for applying calcium stearate to the punch surface and to the die wall face prior to filing the granules in the die of the tableting machine to produce tablets having a weight of 100 mg and thickness of 2.3 mm. The tableting pressure was about 6 kN. About 0.1 % by weight of calcium stearate (lubricant) was adhered to the surface of the tablet obtained.

### Example 9

250 g of captopril (water solubility: about 60 mg/ml), 1460 g of D-mannitol (produced by KYOWA HAKKO KOGYO Co., Ltd./NIKKEN chemical synthetic company, specific surface area: 0.1 m²/g) as used in Example 1 and 90 g of crospovidone ("POLYPLASDON XL-10", produced by ISP) were introduced into a high speed mixing granulator (VG-25 type, produced by POWREX). The mixture was granulated by adding 350 g of purified water. The resulted granules were dried by a fluidized bed drier (FLO-5 type, produced by FREUND Industrial Co., Ltd.) at 80 °C for 15 minutes. The dried granules were passed through a No. 20 wire gauze to give even granules. Subsequently, the granules were subjected to compression molding by a rotary type tableting machine equipped with punches having 8.0 mm Ø flat face and equipped with a device for applying magnesium stearate to the punch surface and to the die wall face prior to filling the granules in the die of the tableting machine to produce tablets having a weight of 180 mg. The thicknesses of the tablets were each 2.95, 3.00 and 3.05 mm, and the tableting pressures were about 11, about 8 and about 6 kN, respectively. About 0.1 % by weight of magnesium stearate (lubricant) was adhered to the surface of the tablet obtained.

### Comparative Example 5

250 g of captopril (water solubility: about 60 mg/ml), 1460 g of sorbitol (TOWA KASEI Co.) and 90 g of crospovidone ("POLYPLASDON XL-10", produced by ISP) were introduced into a high speed mixing granulator (VG-25 type, produced by POWREX). The mixture was granulated by adding 350 g of purified water. The resulted granules were dried by a fluidized bed drier (FLO-5 type, produced by FREUND Industrial Co., Ltd.) at 80°C for 15 minutes. The dried granules were passed through a No. 20 wire gauze to give even granules. Subsequently, the granules were subjected to compression molding by a rotary type tableting machine equipped with a punch having a 8.0 mm Ø flat face and equipped with a device for applying magnesium stearate to the punch surface and to the die wall face prior to filling the granules in the die of the tableting machine to produce tablets having a weight of 180 mg. The thicknesses of the tablets were each 2.95, 3.00 and 3.05 mm, and the tableting pressures were about 11, about 8 and about 6 kN, respectively. About 0.1 % by weight of magnesium stearate (lubricant) was adhered to the surface of the tablet obtained.

### Comparative Example 6

250 g of captopril (water solubility: about 60 mg/ml), 1460 g of white sugar (produced by NITSHIN sugar manufacturing company, Japan) and 90 g of crospovidone ("POLYPLASDON XL-10". produced by ISP) were introduced into a high speed mixing granulator (VG-25 type, produced by POWREX). The mixture was granulated by adding 350 g of purified water. The resulted granules were dried by a fluidized bed drier (FLO-5 type, produced by FREUND Industrial Co., Ltd.) at 80°C for 15 minutes. The dried granules were passed through a No. 20 wire gauze to give even granules. Subsequently, the granules were subjected to compression molding by a rotary type tableting machine equipped with punches having a 8.0 mm ∅ flat face and equipped with a device for applying magnesium stearate to the punch surface and to the die wall face prior to filling the granules in the die of the tableting machine to produce tablets having a weight of 180 mg. The thicknesses of the tablets were each 2.95, 3.00 and 3.05 mm and the tableting pressures were about 11, about 8 and about 6 kN, respectively. About 0.1 % by weight of magnesium stearate (lubricant) was adhered to the surface of the tablet obtained.

### Example 10

250 g of captopril (water solubility: about 60 mg/ml), 1460 g of D-mannitol (produced by KYOWA HAKKO KOGYO Co., Ltd./Nikken Chemical and Synthetic Industry Co., Ltd.; average particle diameter: 30 µm, specific surface area: 0.4 m²/g) and 90 g of crospovidone ("POLYPLASDON XL-10", produced by ISP) were introduced into a high speed mixing granulator (VG-25 type, produced by POWREX). The mixture was granulated by adding 350 g of purified water. The resulted granules were dried by a fluidized bed drier (FLO-5 type, produced by FREUND Industrial Co., Ltd.) at 80°C for 15 minutes. The dried granules were passed through a No. 20 wire gauze to give even granules. Subsequently, the granules were subjected to compression molding by a rotary type tableting machine equipped with punches having a 8.0 mm Ø flat face and equipped with a device for applying magnesium stearate to the punch surface and to the die wall face prior to filling the granules in the die of the tableting machine to produce tablets having a weight of 180 mg. The thicknesses of the tablets were each 2.95, 3.00 and 3.05 mm, and the tableting pressures were about 11, about 8 and about 6 kN, respectively. About 0.1 % by weight of magnesium stearate (lubricant) was adhered to the surface of the tablet obtained.

### Comparative Example 7

250 g of captopril (water solubility: about 60 mg/ml), 1460 g of D-mannitol as used in Example 1 and 90 g of croscalmerose sodium (produced by ASAHI chemical synthetic company, Japan, [Ac-Di-Sol] ) were introduced into ahigh speedmixing granulator (VG-25 type, produced by POWREX). The mixture was granulated by adding 350 g of purified water. The granules were dried by a fluidized bed drier (FLO-5 type, produced by FREUND Industrial Co., Ltd.) at 80°C for 15 minutes. The dried granules were passed through a No. 20 wire gauze to give even granules. Subsequently, the granules were subjected to compression molding by a rotary type tableting machine equipped with punches having a 8.0 mm Ø flat face and equipped with a device for applying magnesium stearate to the punch surface and to the die wall face prior to filling the granules in the die of the tableting machine to produce tablets having a weight of 180 mg. The thicknesses of the tablets were each 2.95, 3.00 and 3.05 mm, and the tableting pressures were about 11, about 8 and about 6 kN, respectively. About 0.1 % by weight of magnesium stearate (lubricant) was adhered to the surface of the tablet obtained.

### Comparative Example 8

250 g of captopril (water solubility: about 60 mg/ml), 1460 g of D-mannitol as used in Example 1 and 90 g of low substituted hydroxypropylcellulose ("L-HPC-LH31", produced by Shin-Etsu Chemical Co., Ltd.) were introduced into a high speed mixing granulator (VG-25 type, produced by POWREX). The mixture was granulated by adding 350 g of purified water. The resulted granules were dried by a fluidized bed drier (FLO-5 type, produced by FREUND Industrial Co., Ltd.) at 80°C for 15 minutes. The dried granules were passed through a No. 20 wire gauze to give even granules. Subsequently, the granules were subjected to compression molding by a rotary type tableting machine equipped with punches having a 8.0 mm Ø flat face and equipped with a device for applying magnesium stearate to the punch surface and to the die wall face prior to filling the granules in the die of the tableting machine to produce tablets having a weight of 180 mg. The thicknesses of the tablets were each 2.95, 3.00 and 3.05, and the tableting pressures were about 11, about 8 and about 6 kN, respectively. About 0.1% by weight of magnesium stearate (lubricant) was adhered to the surface of the tablet obtained.

### Comparative Example 9

250 g of captopril (water solubility: about 60 mg/ml), 1460 g of D-mannitol (produced by KYOWA HAKKO KOGYO Co., Ltd./Nikken Chemical and Synthetic Industry Co., Ltd.; average particle diameter: 7 µm, specific surface area: 1.1 m²/g) and 90 g of crospovidone ("POLYPLASDON XL-10", produced by ISP) were introduced into a high speed mixing granulator (VG-25 type, produced by POWREX). The mixture was granulated by adding 350 g of purified water. The resulted granules were dried by a fluidized bed drier (FLO-5 type, produced by FREUND Industrial Co., Ltd.) at 80°C for 15 minutes. The dried granules were passed through a No. 20 wire gauze to give even granules. Subsequently, the granules were subjected to compression molding by a rotary type tableting machine equipped with punches having a 8.0 mm ∅ flat face and equipped with a device for applying magnesium stearate to the punch surface and to the die wall face prior to filling the granules in the die of the tableting machine to produce tablets having a weight of 180 mg. The thicknesses of the tablets were each 2.95, 3.00 and 3.05 mm, and the tableting pressures were about 11, about 8 and about 6 kN, respectively. About 0.1 % by weight of magnesium stearate (lubricant) was adhered to the surface of the tablet obtained.

The compositions of the tablets are shown in Table 1.

**Table 1**

| | Pharmacologically Active Ingredient | D-mannitol (specific surface area /average particle diameter) | Crospovidone | Lubricant |
|---|---|---|---|---|
| Example 1 | Midodrine 20 g | 1120 g (0.1 m² /g, g µm) | 60 g | EX |
| Example 2 | Midodrine 20 g | 1156 g (0.1 m²/g, 60 µm) | 24 g | EX |
| Example 3 | Midodrine 20 g | 1120 g (0.3 m²/g, 35 µm) | 60 g | EX |
| Example 4 | Midodrine 20 g | 1060 g (0.1 m²/g, g µm) | 120 g | EX |
| Comparative Example 1 | Domperidone 200 g | 2080 g (0.1 m²/g, 60 µm) | 120 g | 0.8 % |
| Comparative Example 2 | None | 2260 g (0.1 m²/g, 60 µm) | 120 g | 1.0 % |

In the table, "EX" indicates that the lubricant is included only on the surface of the tablet by tableting using a tableting machine in which the lubricant has been previously applied to the punch surface and to the die wall face of the tableting machine. In the table, "%" indicates % by weight.

**Table 2**

| | Active Ingredient | D-mannitol (specific surface area /average particle diameter) | Crospovidone | Additive | Lubricant |
|---|---|---|---|---|---|
| Example 5 | 300 g | 1980 g (0.1 m²/g, 60 µm) | 120 g | - | EX |
| Example 6 | 300 g | 1980 g (0.1 m²/g, 60 µm) | 120 g | - | 1.0 % |
| Example 7 | 300 g | 1980 g (0.1 m²/g, 60 µm) | 120 g | - | 0.9 %, EX |

In the table, "Active Ingredient" means the pharmacologically active ingredient, specifically ambroxol hydrochloride, and "EX" indicates that the lubricant is included only on the surface of the tablet by tableting using a tableting machine in which the lubricant has been previously applied to the punch surface and to the die wall face of the tableting machine. In the table, "%" indicates % by weight.

**Table 3**

| | Active Ingredient | D-mannitol (specific surface area /average particle diameter) | Crospovidone | Additive | Lubricant |
|---|---|---|---|---|---|
| Example 8 | 15 g | 1410 g (0.1 m²/g, 60 µm) | 75 g | - | EX |
| Comparative Example 3 | 15 g | - | 75 g | Sorbitol | EX |
| Comparative Example 4 | 15 g | - | 75 g | White sugar 1410 g | EX |

In the table, "Active Ingredient" means the pharmacologically active ingredient, specifically loperamide hydrochloride, and "EX" indicates that the lubricant is included only on the surface of the tablet by tableting using a tableting machine in which the lubricant has been previously applied to the punch surface and to the die wall face of the tableting machine.

**Table 4**

| | Active Ingredient | D-mannitol (specific surface area / average particle diameter) | Disintegrating agent | Additive | Lubricant |
|---|---|---|---|---|---|
| Example 9 | 250 g | 1460 g (0.1 m²/g, 60 µm) | Crospovidone 90 g | - | EX |
| Comparative Example 5 | 250 g | - | Crospovidone 90 g | Sorbitol 1410 g | EX |
| Comparative Example 6 | 250 g | - | Crospovidone 90 g | White sugar 1410 g | EX |
| Example 10 | 250 g | 1460 g (0.4 m²/g, g 30 µm) | Crospovidone 90 g | - | EX |
| Comparative Example 7 | 250 g | 1460 g (0.1 m²/g, 60 µm) | Croscalmerose sodium 90 g | - | EX |
| Comparative Example 8 | 250 g | 1460 g (0.1 m²/g, 60 µm) | Low-hydropropyl-cellulose 90 g | - | EX |
| Comparative Example 9 | 250 g | 1460 g (1.1 m²/g, 7 µm) | Crospovidone 90 g | - | EX |

In the table, "Active Ingredient" means the pharmacologically active ingredient, specifically captopril, and "EX" indicates that the lubricant is included only on the surface of the tablet by tableting using a tableting machine in which the lubricant has been previously applied to the punch surface and to die wall face of the tableting machine.

### Experimental Example

The disintegration time in the oral cavity and the hardness of the tablets in Examples 1 to 10 and Comparative Examples 1 to 7 were determined. The disintegration time was determined as follows. One tablet was orally administered to each of five healthy adults, and the time needed for disintegration only with saliva was measured, based on which the mean value of the disintegration time is calculated. The obtained mean value is defined as the disintegration time. The hardness of the tablet was determined as follows. Five tablets are sampled, and the hardness of the five tablets was measured by a Tablet Hardness tester (produced by Toyama Chemical Co., Ltd.; TH-303 type), based on which the mean value of the hardness of the tablets was calculated. The obtained mean value is determined to be the hardness of the tablet.

The results are shown in the following table.

Meanwhile, in Comparative Examples 1 and 2, the hardness of the tablet obtained does not rise over about 15kN even under a tablet pressure of more than 10kN. Further the occurrence of lamination was observed.

**Table 5**

| Thickness of Tablet | 2.55 mm | 2.6 mm | 2.65 mm |
|---|---|---|---|
| | Hardness : Disintegration time | Hardness : Disintegration time | Hardness : Disintegration time |
| Example 1 | | 32N, 11s | |
| Example 2 | | 33N, 22s | |
| Example 3 | | 39N, 15s | |
| Example 4 | | 32N, 11s | |
| Comparative Example 1 | 18N, 16s | Not molded, - | Not molded, - |
| Comparative Example 2 | 15N, 15s | Not molded, - | Not molded, - |

**Table 6**

| Thickness of Tablet | 2.55 mm | 2.6 mm | 2.65 mm |
|---|---|---|---|
| | Hardness : Disintegration time | Hardness : Disintegration time | Hardness : Disintegration time |
| Example 5 | 60N, 13s | 57N, 13s | 43N, 12s |
| Example 6 | 39N, 33s | 36N, 27s | 20N, 21s |
| Example 7 | 41N, 27s | 39N, 25s | 15N, 22s |

**Table 7**

| | Thickness of Tablet 2.3 mm |
|---|---|
| | Hardness : Disintegration time |
| Example 8 | 51N, 18s |
| Comparative Example 3 | 47N, 125s |
| Comparative Example 4 | 16N, 109s |

**Table 8**

| Thickness of Tablet | 2.95 mm | 3 mm | 3.05 mm |
|---|---|---|---|
| | Hardness Disintegration time | Hardness Disintegration time | Hardness Disintegration time |
| Example 9 | 51N, 15s | 44N, 12s | 30N, 8s |
| Comparative Example 5 | 60N, 185s | 51N, 139s | 42N, 123s |
| Comparative Example 6 | 19N, 103s | 19N, 98s | 12N, 92s |
| Example 10 | 59N, 24s | 54N, 22s | 43N, 19s |
| Comparative Example 7 | 42N, 90s | 31N, 83s | 20N, 72s |
| Comparative Example 8 | 52N, 149s | 49N, 142s | 44N, 138s |
| Comparative Example 9 | 49N, 54s | 60N, 54s | 62N, 63s |

### Example 11

100 g of famotidine (water solubility: about 0.7 mg/ml), 1040 g of D-mannitol (produced by KYOWA HAKKO KOGYO Co., Ltd./Nikken Chemical and Synthetic Industry Co., Ltd.; average particle diameter : 60 µm, specific surface area: 0.1 m²/g) and 60 g of crospovidone ("POLYPLASDON XL-10", produced by ISP) were introduced into a high speed mixing granulator (NSK-250 type, produced by OKADA SEIKO Co., Ltd.). The mixture was kneaded for five minutes after adding 200 g of purified water. The mixture was granulated by an extruding granulator (DGL-1 type, produced by FUJI PAUDAL Co., Ltd.)installed with a 0.8 mm screen 0 and dried by a ventilation box type drier (the Japanese drier of TE-98 type) at 60°C for 60 minutes. The dried granules were passed through a No. 20 wire gauze to give even granules for tableting, Subsequently the granules were subjected to compression molding by a rotary type tableting machine equipped with punches having a 7.0 mm Ø flat face and equipped with a device for applying magnesium stearate to the punch surface and to die wall face prior to filling the granules in the die of the tableting machine to produce tablets having a weight of 120 mg and the thickness of 2.6 mm. The tableting pressure was about 8 kN. About 0.1 % by weight of magnesium stearate (lubricant) was adhered to the surface of the tablet obtained.

The hardness of the obtained tablet was 43N, and the intraorally disintegration time is about 16 seconds.

### INDUSTRIAL APPLICABILITY

According to the present invention, there is provided an intraorally rapidly disintegrable tablet which disintegrates rapidly in the oral cavity without water and has a practical hardness so as not to cause problems such as losing its shape in the process of production and distribution as well as while being handled in a hospital or by patients.

Such an intraorally rapidly disintegrable tablet can be easily taken at an accurate dose even by patients such as aged or infant people who have a weak swallowing strength. Further the tablet can be easily taken without water in the outdoors where water is not available, and accordingly there is advantageous for all patients in that the patients can take medicine as prescribed.

## Claims

1. An intraorally rapidly disintegrable tablet containing a pharmacologically active ingredient with a water solubility of at least 0.5 mg/ml, which is producible by granulating a powdery particle comprising (1) a pharmacologically active ingredient with a water solubility of at least 0.5 mg/ml, (2) D-mannitol in the form of crystals with primary particle average diameter of at least 30 µm and a specific surface area of 0.4 m²/g or less, and (3) crospovidone, followed by compression molding, which further comprises a lubricant included only on the surface of the tablet.

2. The tablet according to claim 1, wherein the pharmacologically active ingredient is a pharmacologically active ingredient with a water solubility of at least 1 µmg/ml.

3. The tablet according to claim 1 or 2, wherein D-mannitol in the form of crystals is D-mannitol in the form of crystals with primary particle average diameter of 30 to 100 µm and a specific surface area of 0.4 to 4 x -10⁻²⁰ m²/g.

4. The tablet according to any one of claims 1 to 3, wherein the lubricant is at least one member selected from the group consisting of magnesium stearate, calcium stearate, stearic acid, stearyl alcohol, stearyl sodium fumarate, sucrose fatty acid ester and talc.

5. The tablet according to any one of claims 1 to 4, which further comprises at least one member selected from the group consisting of a corrigent, a sweetener, a flavor, a coloring agent, a stabilizing agent, an antioxidant, a fluidizing agent and an auxiliary solubilizer.

6. The tablet according to any one of claims 1 to 5, wherein the amount of D-mannitol relative to the whole tablet is in the range of from 20 to 99 % by weight.

7. The tablet according to any one of claims 1 to 6, wherein the amount of crospovidone relative to the whole tablet is in the range of from 0.5 to 30 % by weight.

8. The tablet according to any one of claims 1 to 7, wherein the hardness of the tablet is at least 20 N.

9. The tablet according to any one of claims 1 to 8, which disintegrates in the oral cavity in 30 seconds or less.

10. A method for producing an intraorally rapidly disintegrable tablet containing a pharmacologically active ingredient with a water solubility of at least 0.5 mg/ml, which is **characterized in that** an aqueous medium is added to a powdery particle comprising a pharmacologically active ingredient with a water solubility of at least 0.5 mg/ml, D-mannitol in the form of crystals with primary particle average diameter of at least 30 µm and a specific surface area of 0.4 m²/g or less, and crospovidone; the mixture is kneaded and granulated: the resulting granules are dried; other additives are optionally added to the granules to prepare a material for compression molding; and the material is subjected to compression molding wherein compression molding is carried out by using a compression molding machine, in which a lubricant is previously applied to the punch surface and to the die wall.

11. The method for producing a tablet according to claim 10, wherein the pharmacologically active ingredient is a pharmacologically active ingredient with a water solubility of at least 1 mg/ml.

12. The method for producing a tablet according to claim 10 or 11, wherein the other additives comprise at least one member selected from the group consisting of a corrigent, a sweetener, a flavor, a coloring agent, a fluidizing agent, an antioxidant, a stabilizing agent and an auxiliary solubilizer.

13. The method for producing a tablet according to any one of claims 10 to 12, wherein D-mannitol in the form of crystals is D-mannitol in the form of crystals with primary particle average diameter of 30 to 100 µm and a specific surface area of 0.4 to 4 x 10⁻²⁰ m²/g.

14. The method for producing a tablet according to any one of claims 10 to 13, wherein the lubricant is at least one member selected from the group consisting of magnesium stearate, calcium stearate, stearic acid, stearyl alcohol, stearyl sodium fumarate, sucrose fatty acid ester and talc.

15. The method for producing a tablet according to any one of claims 10 to 14, wherein the amount of D-mannitol relative to the whole tablet is in the range of 20 to 99 % by weight.

16. The method for producing a tablet according to any one of claims 10 to 15, wherein the amount of crospovidone relative to the whole tablet is in the range of 0.5 to 30 % by weight.

17. The method for producing a tablet according to any one of claims 10 to 16, wherein the hardness of the tablet obtained is at least 20 N.

18. The method for producing a tablet according to any one of claims 10 to 17, wherein the tablet disintegrates in the oral cavity in 30 seconds or less.

19. The method for producing a tablet according to any one of claims 10 to 18, wherein the aqueous medium is purified water.

## Patentansprüche

1. Intraoral schnell auflösende Tablette, enthaltend einen pharmakologisch aktiven Bestandteil mit einer Wasserlöslichkeit von mindestens 0,5 mg/ml, die durch Granulieren eines pulverförmigen Teilchens, umfassend (1) einen pharmakologisch aktiven Bestandteil mit einer Wasserlöslichkeit von mindestens 0,5 mg/ml, (2) D-Mannitol in Form von Kristallen mit einem durchschnittlichen Primärteilchendurchmesser von mindestens 30 µm und einer spezifischen Oberfläche von 0,4 m²/g oder weniger und (3) Crospovidon, gefolgt von Pressen, hergestellt werden kann, und die ferner ein Schmiermittel, das nur auf der Oberfläche der Tablette enthalten ist, umfasst.

2. Tablette nach Anspruch 1, wobei der pharmakologisch aktive Bestandteil ein pharmakologisch aktiver Bestandteil mit einer Wasserlöslichkeit von mindestens 1 mg/ml ist.

3. Tablette nach Anspruch 1 oder 2, wobei das D-Mannitol in Form von Kristallen D-Mannitol in Form von Kristallen mit einem durchschnittlichen Primärteilchendurchmesser von 30 bis 100 µm und einer spezifischen Oberfläche von 0,4 bis 4 x 10⁻²⁰ m²/g ist.

4. Tablette nach einem der Ansprüche 1 bis 3, wobei das Schmiermittel mindestens ein Vertreter, ausgewählt aus der Gruppe, bestehend aus Magnesiumstearat, Calciumstearat, Stearinsäure, Stearylalkohol, Stearylnatriumfumarat, Saccharosefettsäureester und Talk, ist.

5. Tablette nach einem der Ansprüche 1 bis 4, die ferner mindestens einen Vertreter, ausgewählt aus der Gruppe, bestehend aus einem Korrigens, einem Süßungsmittel, einem Aromastoff, einem Färbemittel, einem Stabilisator, einem Antioxidationsmittel, einem Fließvermittler und einem zusätzlichen Löslichmacher, umfasst.

6. Tablette nach einem der Ansprüche 1 bis 5, wobei die Menge an D-Mannitol, bezogen auf die gesamte Tablette, im Bereich von 20 bis 99 Gew.-% liegt.

7. Tablette nach einem der Ansprüche 1 bis 6, wobei die Menge an Crospovidon, bezogen auf die gesamte Tablette, im Bereich von 0,5 bis 30 Gew.-% liegt.

8. Tablette nach einem der Ansprüche 1 bis 7, wobei die Härte der Tablette mindestens 20 N beträgt.

9. Tablette nach einem der Ansprüche 1 bis 8, die sich innerhalb von 30 Sekunden oder weniger in der Mundhöhle auflöst.

10. Verfahren zur Herstellung einer sich intraoral schnell auflösenden Tablette, enthaltend einen pharmakologisch aktiven Bestandteil mit einer Wasserlöslichkeit von mindestens 0,5 mg/ml, **dadurch gekennzeichnet, dass** einem pulverförmigen Teilchen, umfassend einen pharmakologisch aktiven Bestandteil mit einer Wasserlöslichkeit von mindestens 0,5 mg/ml, D-Mannitol in Form von Kristallen mit einem durchschnittlichen Primärteilchendurchmesser von mindestens 30 zur und einer spezifischen Oberfläche von 0,4 m²/g oder weniger und Crospovidon, ein wässeriges Medium zugegeben wird, das Gemisch geknetet und granuliert wird, das resultierende Granulat getrocknet wird, dem Granulat gegebenenfalls andere Additive zugegeben werden, um so ein Material zum Pressen herzustellen; und das Material gepresst wird, wobei das Pressen unter Verwendung einer Presse durchgeführt wird, in der vorher ein Schmiermittel auf die Stempeloberfläche und auf die Pressformwand aufgebracht wird.

11. Verfahren zur Herstellung einer Tablette nach Anspruch 10, wobei der pharmakologisch aktive Bestandteil ein pharmakologisch aktiver Bestandteil mit einer Wasserlöslichkeit von mindestens 1 mg/ml ist.

12. Verfahren zur Herstellung einer Tablette nach Anspruch 10 oder 11, wobei die anderen Additive mindestens einen Vertreter, ausgewählt aus der Gruppe, bestehend aus einem Korrigens, einem Süßungsmittel, einem Aromastoff, einem Färbemittel, einem Fließvermittler, einem Antioxidationsmittel, einem Stabilisator und einem zusätzlichen Löslichmacher, umfassen.

13. Verfahren zur Herstellung einer Tablette nach einem der Ansprüche 10 bis 12, wobei das D-Mannitol in Form von Kristallen D-Mannitol in Form von Kristallen mit einem durchschnittlichen Primärteilchendurchmesser von 30 bis 100 µm und einer spezifischen Oberfläche von 0,4 bis 4 x 10⁻²⁰ m²/g ist.

14. Verfahren zur Herstellung einer Tablette nach einem der Ansprüche 10 bis 13, wobei das Schmiermittel mindestens ein Vertreter, ausgewählt aus der Gruppe, bestehend aus Magnesiumstearat, Calciumstearat, Stearinsäure, Stearylalkohol, Stearylnatriumfumarat, Saccharosefettsäureester und Talk, ist.

15. Verfahren zur Herstellung einer Tablette nach einem der Ansprüche 10 bis 14, wobei die Menge an D-Mannitol, bezogen auf die gesamte Tablette, im Bereich von 20 bis 99 Gew.-% liegt.

16. Verfahren zur Herstellung einer Tablette nach einem der Ansprüche 10 bis 15, wobei die Menge an Crospovidon, bezogen auf die gesamte Tablette, im Bereich von 0,5 bis 30 Gew.-% liegt.

17. Verfahren zur Herstellung einer Tablette nach einem der Ansprüche 10 bis 16, wobei die Härte der erhaltenen Tablette mindestens 20 N beträgt.

18. Verfahren zur Herstellung einer Tablette nach einem der Ansprüche 10 bis 17, wobei sich die Tablette innerhalb von 30 Sekunden oder weniger in der Mundhöhle auflöst.

19. Verfahren zur Herstellung einer Tablette nach einem der Ansprüche 10 bis 18, wobei das wässerige Medium gereinigtes Wasser ist.

## Revendications

1. Comprimé à délitement rapide par voie intra-orale contenant un ingrédient actif sur le plan pharmacologique ayant une solubilité dans l'eau d'au moins 0,5 mg/ml, qui peut être produit par granulation d'une particule pulvérulente comprenant (1) un ingrédient actif sur le plan pharmacologique ayant une solubilité dans l'eau d'au moins 0,5 mg/ml, (2) du D-mannitol sous forme de cristaux ayant un diamètre moyen de particule primaire d'au moins 30 µm et une surface spécifique de 0,4 4 m² / g ou moins, et (3) de la crospovidone, puis par moulage par compression, qui comprend en outre un lubrifiant inclus seulement sur la surface du comprimé.

2. Comprimé selon la revendication 1, dans lequel l'ingrédient actif sur le plan pharmacologique est un ingrédient actif sur le plan pharmacologique ayant une solubilité dans l'eau d'au moins 1 mg/ml.

3. Comprimé selon la revendication 1 ou 2, dans lequel le D-mannitol sous forme de cristaux est un D-mannitol sous forme de cristaux ayant un diamètre moyen de particule primaire de 30 à 100 µm et une surface spécifique de 0,4 à 4 x 10⁻²⁰ m²/g.

4. Comprimé selon l'une quelconque des revendications 1 à 3, dans lequel le lubrifiant est au moins un élément choisi dans le groupe constitué par le stéarate de magnésium, le stéarate de calcium, l'acide stéarique, l'alcool stéarylique, le fumarate de stéaryle et de sodium, un ester de saccharose d'acide gras et le talc.

5. Comprimé selon l'une quelconque des revendications 1 à 4, comprenant en outre au moins un élément choisi dans le groupe constitué par un correctif, un édulcorant, une saveur, un agent colorant, un agent stabilisant, un antioxydant, un agent de fluidisation et un agent de solubilisation auxiliaire.

6. Comprimé selon l'une quelconque des revendications 1 à 5, dans lequel la quantité de D-mannitol par rapport au comprimé entier se situe dans la plage allant de 20 % en poids à 99 % poids.

7. Comprimé selon l'une quelconque des revendications 1 à 6, dans lequel la quantité de crospovidone par rapport au comprimé entier se situe dans la plage allant de 0,5 % en poids à 30 % en poids.

8. Comprimé selon l'une quelconque revendication 1 à 7, dans lequel la dureté du comprimé est d'au moins 20 N.

9. Comprimé selon l'une quelconque des revendications 1 à 8, qui se délite dans la cavité orale en 30 secondes ou moins.

10. Procédé de production d'un comprimé à délitement rapide par voie intra-orale contenant un ingrédient actif sur le plan pharmacologique ayant une solubilité dans l'eau d'au moins 0,5 mg/ml, qui est **caractérisé en ce qu'**un milieu aqueux est ajouté à une particule pulvérulente comprenant un ingrédient actif sur le plan pharmacologique ayant une solubilité dans l'eau d'au moins 0,5 mg/ml, du D-mannitol sous forme de cristaux ayant un diamètre moyen de particule primaire d'au moins 30 µm et une surface spécifique de 0,4 m²/g ou moins, et de la crospovidone ; le mélange est malaxé et soumis à une granulation ; les granulés résultants sont séchés ; d'autres additifs sont facultativement ajoutés aux granulés pour préparer une matière pour moulage par compression ; et la matière est soumise à un moulage par compression, dans lequel le moulage par compression est réalisé en utilisant une machine de moulage par compression dans laquelle un lubrifiant est préalablement appliqué sur la surface du poinçon et sur la paroi de la filière.

11. Procédé de production d'un comprimé selon la revendication 10, dans lequel l'ingrédient actif sur le plan pharmacologique est un ingrédient actif sur le plan pharmacologique ayant une solubilité dans l'eau d'au moins 1 mg/ml.

12. Procédé de production d'un comprimé selon la revendication 10 ou 11, dans lequel les autres additifs comprennent au moins un élément choisi dans le groupe constitué par un correctif, un édulcorant, une saveur, un agent colorant, un agent de fluidisation, un antioxydant, un agent stabilisant et un agent de solubilisation auxiliaire.

13. Procédé de production d'un comprimé selon l'une quelconque des revendications 10 à 12, dans lequel le D-mannitol sous forme de cristaux est un D-mannitol sous forme de cristaux ayant un diamètre moyen de particule primaire de 30 à 100 µm et une surface spécifique de 0,4 à 4 x 10⁻²⁰ m²/g_{.}

14. Procédé de production d'un comprimé selon l'une quelconque des revendications 10 à 13, dans lequel le lubrifiant est au moins un élément choisi dans le groupe constitué par le stéarate de magnésium, le stéarate de calcium, l'acide stéarique, l'alcool stéarylique, le fumarate de stéaryle et de sodium, un ester de saccharose d'acide gras et le talc.

15. Procédé de production d'un comprimé selon l'une quelconque des revendications 10 à 14, dans lequel la quantité de D-mannitol par rapport au comprimé entier se situe dans la plage allant de 20 % en poids à 99 % poids.

16. Procédé de production d'un comprimé selon l'une quelconque des revendications 10 à 15, dans lequel la quantité de crospovidone par rapport au comprimé entier se situe dans la plage allant de 0,5 % en poids à 30 % en poids.

17. Procédé de production d'un comprimé selon l'une quelconque des revendications 10 à 16, dans lequel la dureté du comprimé obtenu est d'au moins 20 N.

18. Procédé de production d'un comprimé selon l'une quelconque des revendications 10 à 17, dans lequel le comprimé se délite dans la cavité orale en 30 secondes ou moins.

19. Procédé de production d'un comprimé selon l'une quelconque des revendications 10 à 18, dans lequel le milieu aqueux est de l'eau purifiée.
